# EUROPEAN PATENT SPECIFICATION

(11) **EP 1 398 004 B1**
(45) Date of publication and mention of the grant of the patent: **13.08.2008**
(21) Application number: 03020413.5
(22) Date of filing: 10.09.2003
(51) Int. Cl.: A61B 19/00

(54) **Cleaning device for medical instrument**
Reinigungsvorrichtung für medizinische Geräte
Dispositif de nettoyage pour instruments médicaux

(30) Priority: 13.09.2002 JP 2002268730
(43) Date of publication of application: 17.03.2004
(73) Proprietor: Olympus Corporation, Shibuya-ku, Tokyo (JP)
(72) Inventor: Sasaki, Katsumi, Olympus Optical Co., Ltd, Hachioji-shi Tokyo (JP); Masuda, Shinya, Olympus Optical Co., Ltd, Hachioji-shi Tokyo (JP); Murakami, Eiji, Olympus Optical Co., Ltd, Hachioji-shi Tokyo (JP); Masubuchi, Ryoji, Olympus Optical Co., Ltd, Hachioji-shi Tokyo (JP)
(74) Representative: von Hellfeld, Axel

(56) References cited:
- EP-A- 0 638 296
- US-A- 5 288 467
- US-A1- 2002 165 469
- PATENT ABSTRACTS OF JAPAN vol. 2000, no. 22, 9 March 2001 (2001-03-09) -& JP 2001 120643 A (FUJI PHOTO OPTICAL CO LTD), 8 May 2001 (2001-05-08)

## Description

The present invention relates to a device for cleaning medical instruments including treatment portions in insertion portions.

In Jpn. Pat. Appln. KOKAI Publication No. 2000-296135, an ultrasonic treatment instrument has been proposed as a surgical treatment instrument. In general, the ultrasonic treatment instrument includes a handle unit including an insertion portion, a probe unit attached to the handle unit, and a vibrator unit for driving a probe. A jaw which is an open/close member for grasping the ultrasonic treatment instrument is attached to a tip end of the insertion portion of the handle unit. This jaw is connected to an operation rod inserted in a channel formed in the insertion portion of the handle unit, and is rotated/operated, when the operation rod is moved forwards/backwards by a handle of the handle unit. A probe of the probe unit is inserted through the insertion portion of the handle unit, and the distal-end portion of the probe is disposed opposite to the jaw. Moreover, a tissue is treated by the distal-end portion of the probe and the jaw.

Since this type of ultrasonic treatment instrument is a medical instrument, the instrument needs to be cleaned every use. To clean the ultrasonic treatment instrument, simple cleaning methods such as brush cleaning and shower cleaning in running water have heretofore been carried out.

In this conventional cleaning method, however, especially a cleaning efficiency of a treatment portion including a complicated structure is deteriorated, a lot of trouble and much time are required for cleaning the treatment portion, and efficiency of a cleaning operation of an endoscope has been bad.

Examples of cleaning devices and techniques known for use in fields such as dentistry are described in European Patent Application Publication No. 0 638 296 A1, United States Patent No. 5,961,937 and United States Patent No. 5,090,433. None of these cleaning devices, however, provides an arrangement which optimizes both ease of use as well as care for the instruments to be cleaned.

An object of the present invention is to provide a cleaning device for a medical instrument, capable of efficiently cleaning a treatment portion of the medical instrument which easily becomes dirty.

The present invention provides a cleaning device having the features recited in claim 1. Preferred features of the invention are recited in the dependent claims.

According to an aspect of the present invention, there is provided a cleaning device which cleans a treatment portion of a medical instrument, comprising:
a cleaning cover including a containing portion which surrounds the treatment portion and which contains the treatment portion, and an insertion opening via which the treatment portion is inserted into the containing portion;
an engagement portion which engages with the medical instrument including the treatment portion inserted in the containing portion via the insertion opening and which holds the medical instrument in the cleaning cover;
a water supply port which is disposed in the cleaning cover and which communicates with the containing portion and which supplies cleaning water into the containing portion; and
a drain port which is disposed in the cleaning cover and which communicates with the containing portion and via which the cleaning water in the containing portion is drained to the outside of the cleaning cover.

The invention can be more fully understood from the following detailed description when taken in conjunction with the accompanying drawings, in which:
FIG. 1 is a side view of an assembled state of an ultrasonic treatment instrument;
FIG. 2 is a side view of a handle unit of the ultrasonic treatment instrument;
FIG. 3A is a side view of a probe unit;
FIG. 3B is a side view vibrator unit of the ultrasonic treatment instrument;
FIG. 4A is a vertical sectional view of an insertion sheath portion in the handle unit of the ultrasonic treatment instrument;
FIG. 4B is a transverse sectional view of the insertion sheath portion in the handle unit along a line 4A-4A in FIG. 4A;
FIG. 4C is a vertical sectional view of the insertion sheath portion in the handle unit in the vicinity of a part 4C in FIG. 4A;
FIG. 5 is a vertical sectional view of an operation portion in the handle unit of the ultrasonic treatment instrument;
FIG. 6 is a side view of a whole cleaning device according to the first embodiment of the present invention;
FIG. 7A is a vertical sectional view of the cleaning device;
FIG. 7B is a transverse sectional view of a part along line 7B-7B in FIG. 7A;
FIG. 8A is a vertical sectional view in a state in which the cleaning device is attached to the ultrasonic treatment instrument;
FIG. 8B is a transverse sectional view of a part along line 8B-8B in FIG. 8A;
FIG. 9 is a side view of the whole cleaning device according to a second embodiment of the present invention;
FIG. 10 is a vertical sectional view in a use state of the cleaning device according to the second embodiment;
FIG. 11 is a side view of the whole cleaning device according to a third embodiment of the present invention;
FIG. 12 is a vertical sectional view in a use state of the cleaning device according to a third embodiment;
FIG. 13 is a side view of the whole cleaning device according to a fourth embodiment of the present invention;
FIG. 14 is a rear view of the cleaning device according to the fourth embodiment;
FIG. 15 is a vertical sectional view in the use state of the cleaning device according to the fourth embodiment; and
FIG. 16 is a transverse sectional view in the use state of the cleaning device according to the fourth embodiment.

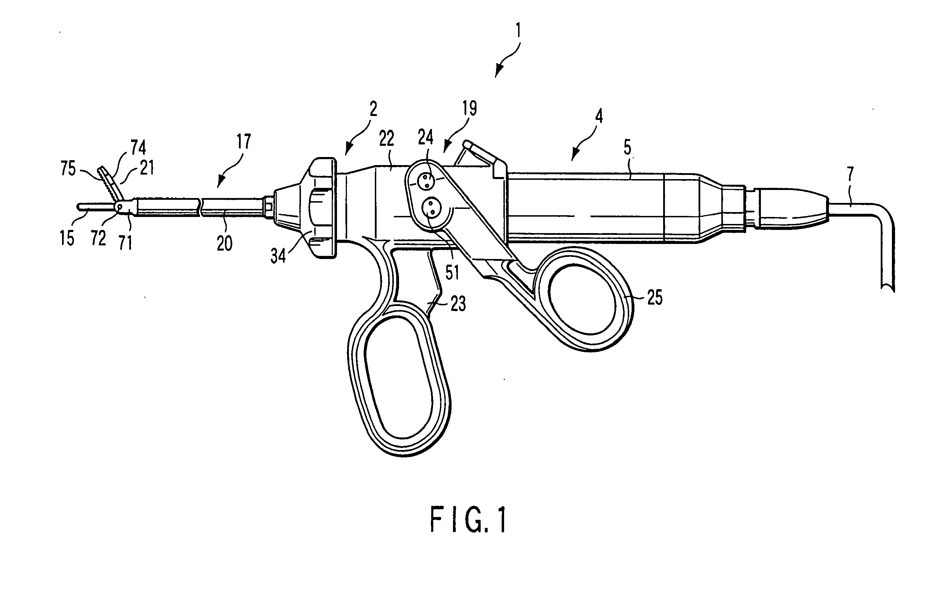

With reference to FIGS. 1 to 8, for example, a cleaning device for a surgical treatment instrument according to a first embodiment of the present invention will be described as a medical device.

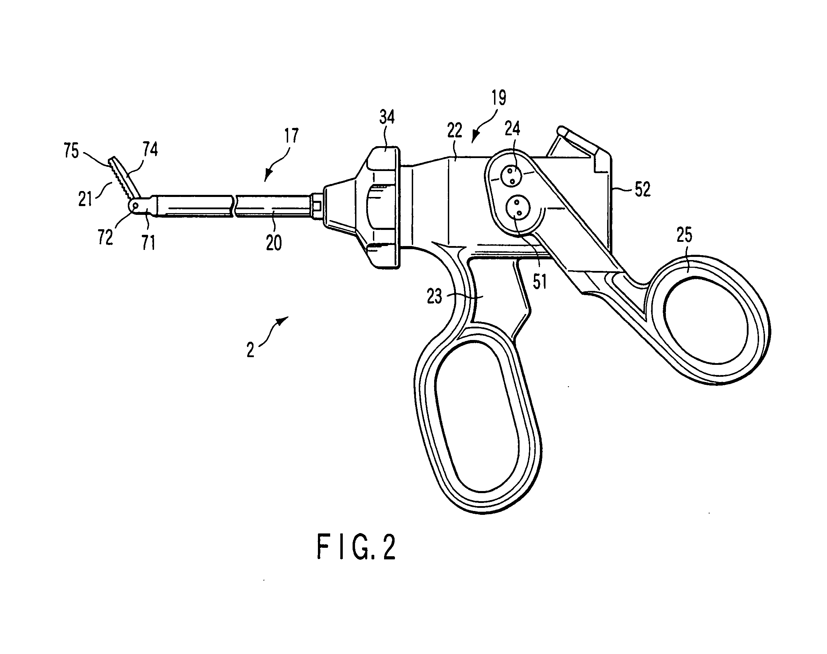

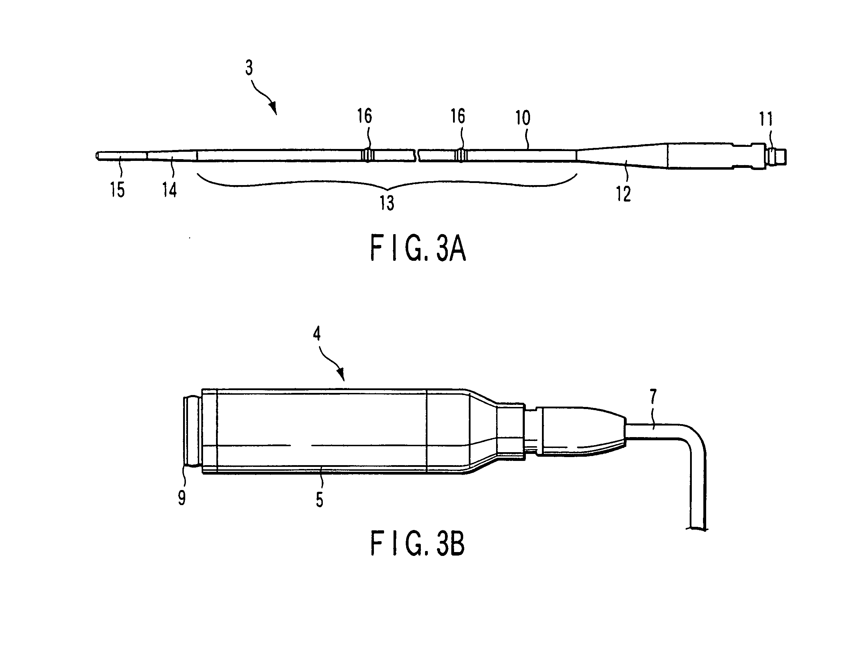

The surgical treatment instrument which is a cleaning object in the present embodiment is an ultrasonic treatment instrument 1. The ultrasonic treatment instrument 1 includes a handle unit 2 shown in FIG. 2, a probe unit 3 shown in FIG. 3A, and a vibrator unit 4 shown in FIG. 3B. When the ultrasonic treatment instrument 1 is used, the respective units 2, 3, 4 are assembled as shown in FIG. 1.

As shown in FIG. 3B, the vibrator unit 4 includes a cylindrical exterior cover 5, and an ultrasonic vibrator and horn (not shown) are disposed in the exterior cover 5. A hand piece cord 7 to be electrically connected to a power supply (not shown) is connected to a base end of the vibrator unit 4. A power for driving is supplied to the ultrasonic vibrator from the power supply via the hand piece cord 7 to drive the ultrasonic vibrator. A connection portion 9 to be connected to the handle unit 2 is formed in a front peripheral edge of the vibrator unit 4.

As shown in FIG. 3A, the probe unit 3 includes a rod-shaped vibration transmission member 10, and ultrasonic vibration generated in the ultrasonic vibrator of the vibrator unit 4 is transmitted to a distal-end treatment portion by this rod-shaped vibration transmission member 10. A male screw 11 to be screwed in a female screw formed in the tip end of the horn is formed in a proximate-end position of the vibration transmission member 10. Furthermore, the vibration transmission member 10 includes: a conical proximate-end horn 12 for enlarging an amplitude of the ultrasonic vibration amplified by the horn on the side of the vibrator unit 4 further into a second stage; a middle portion 13 positioned on the side of a tip end of the proximate-end horn 12; a conical distal-end horn 14, positioned on the side of the tip end of the middle portion 13, for enlarging the amplitude of the ultrasonic vibration amplified by the proximate-end horn 12 into a final stage; and a cylindrical tip end 15 positioned on the side of the tip end of the distal-end horn 14. The ultrasonic vibration generated in the ultrasonic vibrator is gradually amplified by the horn of the vibrator unit 4, the proximate-end horn 12 in the vibration transmission member 10, and the distal-end horn 14, and is transmitted to the tip end 15 of the vibration transmission member 10. In the middle portion 13 of the vibration transmission member 10, a plurality of flange-shaped support members 16 are positioned in second and subsequent nodes on the side of the tip end of the transmitted ultrasonic vibration.

As shown in FIG. 2, the handle unit 2 includes an operation portion 19, an insertion sheath portion 17 including a longitudinal overcoat tube 20 rotatably attached to the operation portion 19, and a distal-end function portion (treatment portion) 21 disposed in the tip end of the insertion sheath portion 17. The operation portion 19 of the handle unit 2 includes an operation portion main body 22, a fixed handle 23 formed integrally with the operation portion main body 22, and a movable handle 25 rotatably attached to the operation portion main body 22 via a handle support shaft 24.

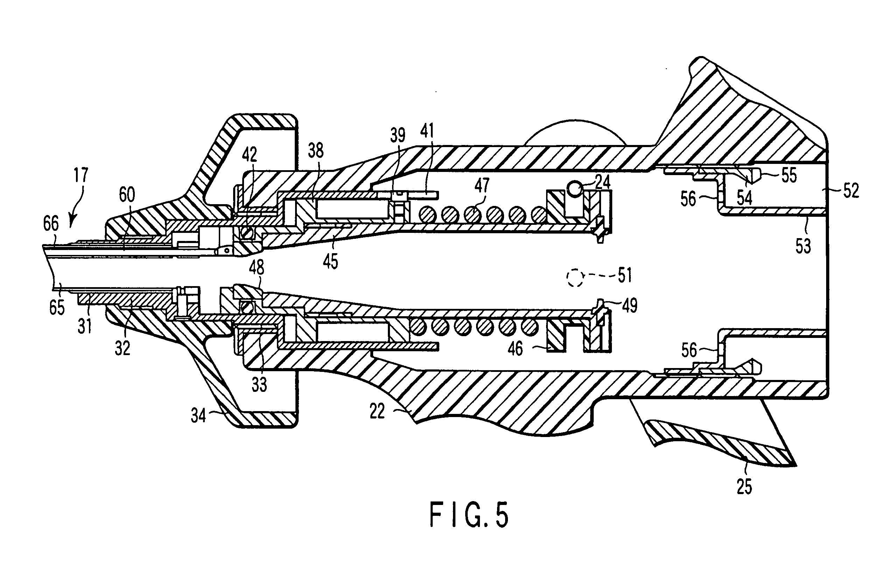

As shown in FIG. 5, a cylindrical joint member 32 disposed in the operation portion main body 22 via a pipe fixing member 31 is fixed/connected to the proximate-end portion of the insertion sheath portion 17. The joint member 32 is attached to the operation portion main body 22 by a fixing ring 33 so as to rotatable around an insertion portion axis. A rotary operation knob 34 is fixed to the joint member 32. When the rotary operation knob 34 is rotated/operated, the insertion sheath portion 17 can be rotated/operated around a sheath axis.

A cylindrical driving force transmission intermediate member 38 to be connected to an operation rod 60 described later is fitted in the joint member 32 so as to be movable in an insertion portion axial direction. A pin 39 is attached to a rear-end portion of the driving force transmission intermediate member 38. This pin 39 is fitted/engaged in a guide groove 41 which is cut/formed in the joint member 32 so as to extend in the insertion portion axial direction. This regulates the rotation of the driving force transmission intermediate member 38 and allows only the movement into the insertion portion axial direction. An O ring 42 is fitted around the outer periphery of the distal-end portion of the driving force transmission intermediate member 38, and the O ring 42 is disposed between the driving force transmission intermediate member 38 and joint member 32.

Moreover, as shown in FIG. 5, a distal-end outer peripheral portion of a slider receiver member 45 is screwed in the driving force transmission intermediate member 38, and both the portion and the member are coaxially assembled/fixed. A slider member 46 is slidably fitted in the outer periphery of the driving force transmission intermediate member 38 on the side of the rear end. A coiled spring 47 is wound around the outer periphery of the slider receiver member 45 between the driving force transmission intermediate member 38 and the slider member 46.

A first packing 48 in the form of the O ring is attached to the inner surface of the driving force transmission intermediate member 38. When the probe unit 3 is attached to the handle unit 2, the first packing 48 is bonded to the outer periphery of the vibration transmission member 10 of the probe unit 3 to hold the vibration transmission member 10 and to close a gap between the units. A second packing 49 having the O-ring shape is also attached to a hand-side end of the slider receiver member 45. When the probe unit 3 is attached to the handle unit 2, the second packing 49 is bonded to the outer periphery of the vibration transmission member 10 of the probe unit 3 to hold the vibration transmission member 10 and to close the gap between the units.

Since the packings 48, 49 are disposed in this manner, for example, aeroperitoneum gas at the time of a surgical operation under an abdominoscope can be prevented from passing through an inner gap from an insertion portion tip end and leaking. Since two packings 48, 49 are both formed of a rubber, and elastic, the vibration transmission member 10 to ultrasonically vibrate is steadily supported without any difficulty.

An engaging pin 51 to engage with the slider member 46 in the operation portion main body 22 is disposed in the movable handle 25 of the handle unit 2. When the movable handle 25 is rotated centering on the handle support shaft 24, the movement of the movable handle 25 is transmitted to the slider member 46 via the engaging pin 51. Since the helical compression spring 47 is constantly attached in a compressed/urged state, a certain or more operation force applied to the movable handle 25 in a closing direction is absorbed by deformation of the helical compression spring 47. Accordingly, the slider receiver member 45 is slid/operated in a state in which an overload applied to the slider receiver member 45 is prevented.

As shown in FIG. 5, a vibrator connection portion 52 for detachably attaching the vibrator unit 4 is disposed in a rear-end portion of the handle unit 2 in the operation portion main body 22. The vibrator connection portion 52 includes a cylindrically formed frame member 53 and a cylindrical connection member 54. The frame member 53 and connection member 54 are screwed/fixed into the inner surface of the operation portion main body 22. A screwed/fixed portion of the frame member 53 is positioned before the connection member 54. The rear-end portion of the frame member 53 is concentrically disposed inside the connection member 54. When the vibrator unit 4 is attached to the handle unit 2, a front-end portion of the cylindrical exterior cover 5 of the vibrator unit 4 is fitted between the frame member 53 and connection member 54. The connection portion 9 of the vibrator unit 4 is fitted into/engaged with a distal-end engagement portion 55 of the frame member 53, so that the vibrator unit 4 is fixed to the handle unit 2. The connection portion 9 is formed, for example, by a C ring so as to swell outwards. A plurality of through-holes 56 are appropriately scattered and disposed in a wall portion of the frame member 53.

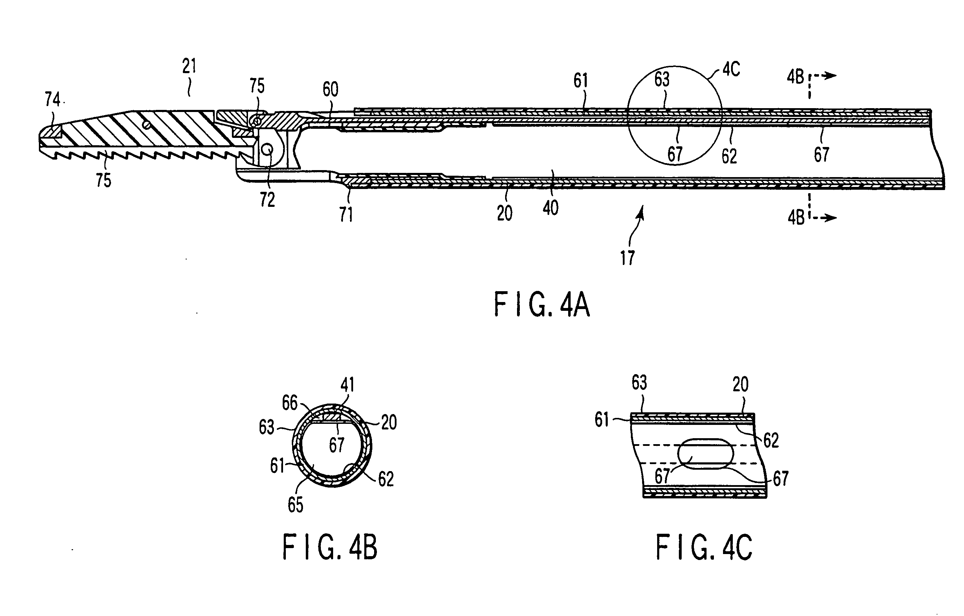

Next, the insertion sheath portion 17 of the handle unit 2 will be described. As shown in FIG. 4A, the overcoat tube 20 of the insertion sheath portion 17 includes an outer pipe (overcoat tube) 61 and an inner pipe 62, and the outer periphery of the outer pipe 61 is subjected to an electric insulating treatment by an insulating tube 63. In the electric insulation, the insertion sheath portion 17 may also be subjected to treatment means such as coating. As shown in FIG. 4B, the outer pipe 61 includes a main channel 65 for passing the vibration transmission member 10 of the probe unit 3, and a sub-channel 66 for passing the operation rod 60. Similarly, as shown in FIG. 4B, for the inner pipe 62 forming the main channel 65, a part of a circular sectional portion is crushed to be flat to form a gap as the sub-channel 66 between the section and the inner surface of the outer pipe 61. A through-hole 67 is formed in one or more appropriate portions of the inner pipe 62 disposed opposite to the sub-channel 66, so that the main channel 65 can be connected to the sub-channel 66.

As shown in FIG. 4A, the distal-end function portion 21 of the handle unit 2 includes a distal-end chip member 71 attached to the distal-end portion of the outer pipe 61 and formed, for example, of a metal, and a single swing hold member 74 rotatably attached to the distal-end chip member 71 via a support shaft 72. This hold member 74 holds a grasp member 75. The tip end of the operation rod 60 is connected to the proximate-end portion of the hold member 74 via a support shaft 76. When the movable handle 25 is rotated to move the operation rod 60 forwards/backwards, the hold member 74 rotates, and an operation of opening/closing the distal-end function portion 21 is carried out. When the distal-end function portion 21 is disposed opposite to the distal-end portion 15 of the probe unit 3 in the vibration transmission member 10, and a living tissue of a treatment object is grasped by both the portions, a vibration energy is transmitted to the living tissue from the vibration transmission member 10.

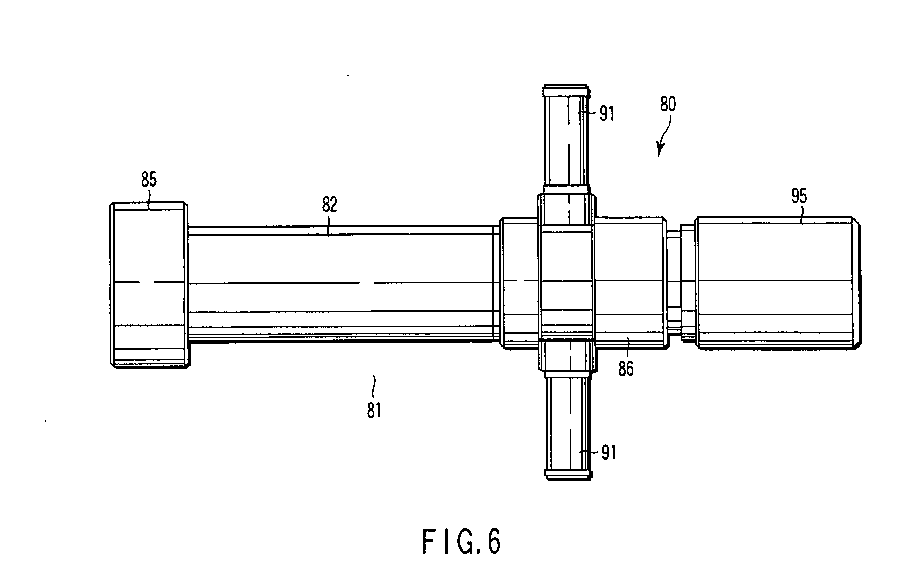

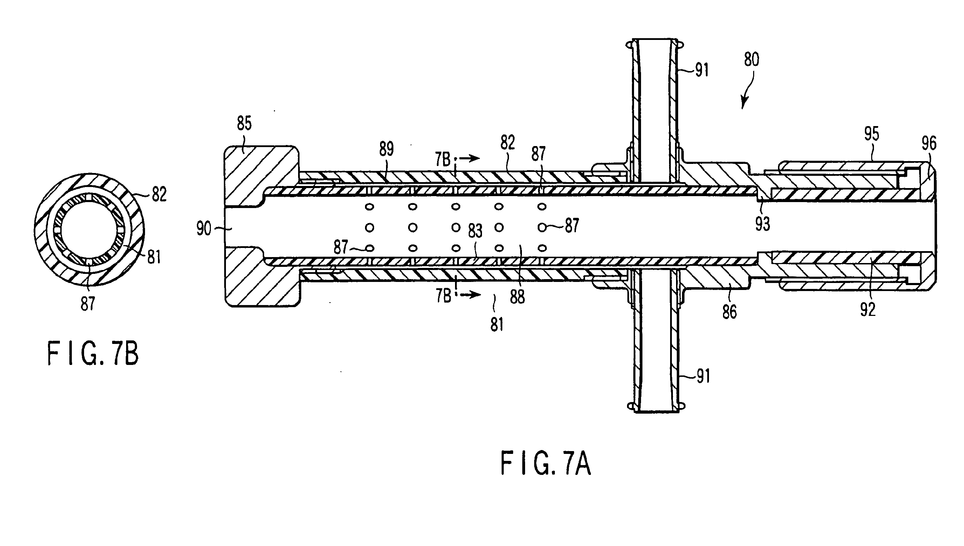

Next, a cleaning device 80 for cleaning the ultrasonic treatment instrument 1 will be described. As shown in FIGS. 6 and 7, the cleaning device 80 includes a cleaning cover 81 which has an elongated shape. For the cleaning cover 81, as shown in FIG. 7, an outer pipe 82 and inner pipe 83 are concentrically disposed and constituted in a double tube form, and a cylindrical member is constituted which can surround and contain a cleaning object portion of the ultrasonic treatment instrument 1. Both tip ends of the outer pipe 82 and inner pipe 83 are detachably fixed to a distal-end member 85 constituted of a cylindrical member including a relatively thick wall. Both rear ends of the outer pipe 82 and inner pipe 83 are detachably fixed to a nozzle member 86 constituted of a cylindrical member including a relatively thick wall. A peripheral edge in the tip end of the outer pipe 82 is screwed in a rear edge of the distal-end member 85. A peripheral edge in the tip end of the nozzle member 86 is screwed in a rear-end outer peripheral edge of the outer pipe 82. The outer pipe 82 is connected to the distal-end member 85 in a liquid-tight manner. The outer pipe 82 is also connected to the nozzle member 86 in the liquid-tight manner. The distal-end portion of the inner pipe 83 is fitted into the distal-end member 85 in the liquid-tight manner. The rear-end portion of the outer pipe 82 is fitted into the nozzle member 86 in the liquid-tight manner.

The outer pipe 82 and inner pipe 83 are formed by transparent materials so that the inside can be seen through. The distal-end member 85 and nozzle member 86 may also be formed of the transparent materials, but are formed of metals and are opaque herein. In the present specification, the nozzle does not mean that the nozzle is limited to the metal material.

In the wall portion in the intermediate portion of the inner pipe 83, a plurality of jet ports (water supply nozzle ports) 87 are arranged at equal intervals in a circumferential direction of the pipe, and a plurality of arrays of jet ports 87 are arranged and formed in a pipe axial direction. A region in the inner pipe 83 where a plurality of jet ports 87 are arranged forms a cleaning region (containing portion) 88 for containing/arranging the cleaning object of the ultrasonic treatment instrument 1. A gap is made in the outer pipe 82 and inner pipe 83, and this gap forms a supply channel 89 for distributing the cleaning water to the respective jet ports 87.

An inner hole of the distal-end member 85 forms a drain port 90 leading into the cleaning cover 81. The cleaning water in the cleaning cover 81 is drained to the outside by the drain port 90.

One or more supply nozzles 91 are attachment the nozzle member 86. The cleaning water is supplied to the supply channel 89 via the respective supply nozzles 91. Two water supply tubes (not shown) connected to a water supply such as a water supply tank and pump incorporated in the cleaning machine for the treatment instrument can separately be connected to each supply nozzle 91.

Fixing means, attached to the distal-end portion of the surgical treatment instrument, for holding the cleaning cover 81 is disposed in the rear-end portion of the nozzle member 86. The inner hole of the rear-end portion of the nozzle member 86 constitutes an insertion port for inserting the distal-end portion of the surgical treatment instrument. An elastic tube 92 is fitted into the inner hole of the rear-end portion of the nozzle member 86. In the nozzle member 86, an abutment portion 93 for receiving an inner end of the elastic tube 92 is formed in an inner end of a region in which the elastic tube 92 is fitted. A push-in ring 95 is screwed into the rear-end portion of the nozzle member 86. A flange 96 of the push-in ring 95 is brought into contact with an outer end of the elastic tube 92, and the push-in ring 95 is screwed into the nozzle member 86. Then, the elastic tube 92 is pushed in the axial direction, and the elastic tube 92 is compressed in the axial direction, so that an inner diameter of the elastic tube 92 can be reduced. An engagement portion is constituted to engage with the portion of the medical instrument 1 inserted via the insertion port constituted of the nozzle member 86 and to hole the medical instrument 1 in the cleaning cover 81.

Next, a procedure and function will be described in a case in which the cleaning device 80 of the present embodiment is used to clean the ultrasonic treatment instrument 1.

The handle unit 2, treatment probe, and generation unit are assembled to use the ultrasonic treatment instrument 1 in the state shown in FIG. 1. Moreover, when the ultrasonic treatment instrument 1 is used, the ultrasonic treatment instrument 1 becomes dirty. Therefore, the instrument needs to be cleaned every use.

Rough dirt on the surface of the ultrasonic treatment instrument 1 can quickly be removed to a certain degree even by the shower cleaning by the treatment instrument cleaning machine or the exposure to the running water. However, the treatment portion such as the distal-end function portion 21 includes concave/convex portions and gaps. Since the portion has an especially complicated shape, degree of adhesion of dirt is strong. To remove the dirt from this portion, the cleaning efficiency is bad only with the shower cleaning or the exposure to the running water.

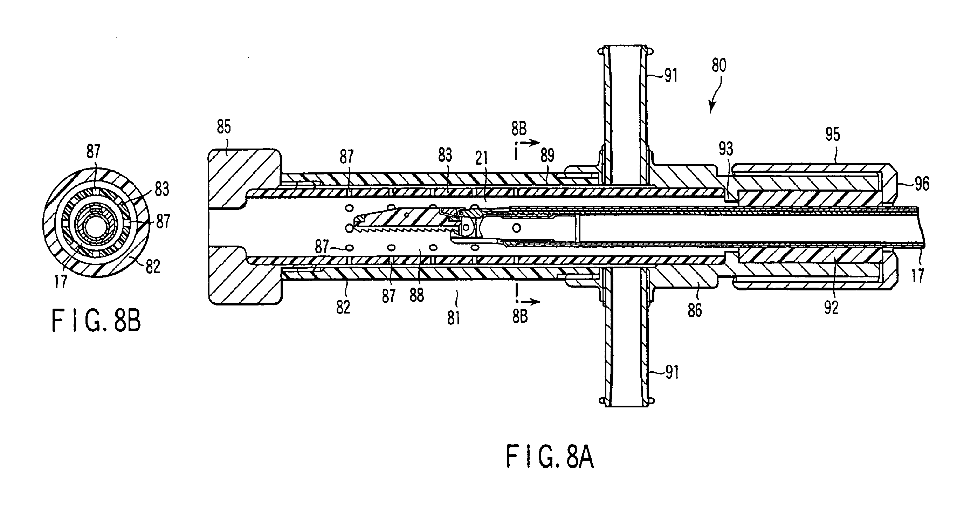

To solve the problem, the cleaning device 80 of the present embodiment is used to remove the dirt of the distal-end function portion 21. To clean the ultrasonic treatment instrument 1, in a state in which the probe unit 3 is removed from the handle unit 2 of the ultrasonic treatment instrument 1 as shown in FIG. 8, the distal-end portion of the handle unit 2 in the insertion sheath portion 17 is covered with the cleaning cover 81, and the device is attached. That is, as shown in FIGS. 7A and 7B, the distal-end portion of the insertion sheath portion 17 of the handle unit 2 from which the probe unit 3 has been removed is inserted into the cleaning cover 81 of the cleaning device 80 to position the distal-end function portion (treatment portion) 21 in the cleaning region 88.

Next, as shown in FIGS. 8A and 8B, the push-in ring 95 is screwed in, the elastic tube 92 is compressed in the axial direction by the flange 96 of the push-in ring 95, and the inner diameter of the elastic tube 92 is reduced. Then, the elastic tube 92 tightens the outer periphery of the insertion sheath portion 17 inserted into the tube, and the cleaning cover 81 is fixed to the insertion sheath portion 17. Since the insertion sheath portion 17 is elastically fastened by the elastic tube 92 in this manner, an outer surface treatment layer of the insertion sheath portion 17 protected by the insulating tube 63 can be prevented from being damaged.

Subsequently, supply tube line means such as a water supply tube connected to the water supply is individually connected to the respective supply nozzles 91. As the water supply, it is possible to use the water supply tank or pump incorporated in the cleaning machine for the treatment instrument in which the treatment instruments such as the ultrasonic treatment instrument are disposed to carry out the shower cleaning or running water cleaning. The water supply tube (not shown) connected to the water supply is connected to the supply nozzle 91.

Moreover, the high-pressure cleaning water pressurized from the water supply through the water supply tube is distributed to the jet ports (water supply nozzle ports) 87 through the supply passage 89, and the high-pressure cleaning water from the periphery is spouted toward the distal-end function portion (treatment portion) 21 positioned in the cleaning region 88 from the respective jet ports 87. Since the high-pressure cleaning water is vigorously spouted onto the distal-end function portion 21 from the periphery, solid dirt can be removed, and further every corner can securely be cleaned without any unevenness. Since the cleaning water is supplied to the supply channel 89 from a plurality of supply nozzles 91, the high-pressure cleaning water can uniformly be distributed to the respective jet ports 87, and all the circumference can strongly be cleaned.

In the cleaning operation, the ultrasonic treatment instrument 1 together with the attached cleaning device 80 is installed in a cleaning tank of the cleaning machine or a cleaning tray of a cleaning unit, and an exterior portion of the ultrasonic treatment instrument 1 is preferably subjected to the shower cleaning or exposed to the running water and is also cleaned.

When connected portions of screwed portions are disconnected, the respective components of the cleaning device 80 can all be disassembled. Especially, the distal-end member 85, nozzle member 86, outer pipe 82, and inner pipe 83 can easily be disassembled, when the distal-end member 85 and nozzle member 86 are removed from the outer pipe 82. Therefore, the components of the cleaning device 80 can also easily be cleaned.

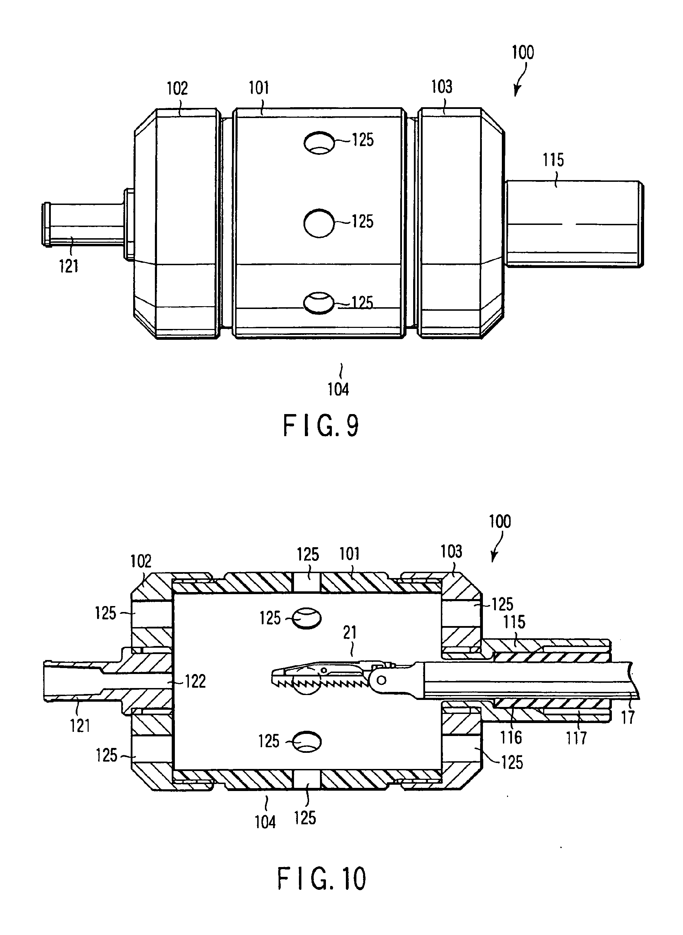

The cleaning device according to a second embodiment of the present invention will be described with reference to FIGS. 9 and 10. In the present embodiment, the same components as those of the first embodiment are denoted with the same reference numerals, and the detailed description is omitted.

In a cleaning device 100 according to the present embodiment, a front-end plate 102 and rear-end plate 103 are attached to front and rear ends of a cylindrical member 101 to close openings of the cylindrical member 101. Accordingly, a cleaning cover 104 is constituted to surround the cleaning region. The front-end plate 102 and rear-end plate 103 are screwed into and attached to the front-end and rear-end portions of the cylindrical member 101 so as to be attachable/detachable.

Fixing means which engages with the distal-end portion of the surgical treatment instrument to hole the cleaning cover 104 in the surgical treatment instrument is disposed in the rear-end plate 103. The fixing means includes a hold cylinder 115 for passing the insertion sheath portion 17 of the ultrasonic treatment instrument 1, the hold cylinder 115 is disposed coaxially with the cleaning cover 104, and the insertion sheath portion 17 of the ultrasonic treatment instrument 1 is passed through the cylinder. A front end of the hold cylinder 115 is screwed into the rear-end plate 103, and the hold cylinder is fixed to the rear-end plate 103 so as to be attachable/detachable.

As shown in FIG. 10, an elastic tube 116 is fitted into the hold cylinder 115. A part of the outer periphery of the elastic tube 116 is meshed with a screw portion 117 formed in the inner surface of the hold cylinder 115, and is bonded if necessary. Accordingly, the elastic tube 116 is fixed to the hold cylinder 115. When the insertion sheath portion 17 of the ultrasonic treatment instrument 1 is inserted through the hold cylinder 115, the elastic tube 116 contacts the outer periphery of the insertion sheath portion 17 with pressure, and engages with the insertion sheath portion 17.

A supply nozzle 121 is disposed coaxially with the cleaning cover 104 in the front-end plate 102 of the cleaning cover 104, screwed into the front-end plate 102, and accordingly attached. The supply tube line means such as the water supply tube connected to the water supply is connected to the supply nozzle 121. In an inner end of the supply nozzle 121, a water supply port 122 is formed and opened toward the cleaning region in the cleaning cover 104. The supply nozzle 121 and hold cylinder 115 are arranged on a central axis of the cleaning cover 104, and a hold axis by the hold cylinder 115 is constituted so as to agree with a jet direction of the water supply port 122.

Drain ports 125 are formed in the cylindrical member 101, front-end plate 102, and rear-end plate 103 of the cleaning cover 104, respectively, and the cleaning water which has overflowed in the cleaning region in the cleaning cover 104 is drained to the outside of the cleaning cover 104 via the drain ports 125.

When the cleaning device 100 according to the present invention is used, in the same manner as in the first embodiment, the distal-end portion of the insertion sheath portion 17 in the handle unit 2 from which the probe unit 3 has been removed is inserted into the hold cylinder 115 to position the distal-end function portion (treatment portion) 21 in the cleaning region in the cleaning cover 104.

Through the supply tube line means such as the water supply tube connected to the water supply, the high-pressure cleaning water is directed toward the cleaning region in the cleaning cover 104 from the water supply port 122 opened in an inner end of the supply nozzle 121 and spouted onto the distal-end function portion 21. Since the high-pressure cleaning water is vigorously spouted onto the distal-end function portion 21, even greasy dirt is removed. The cleaning water which has overflowed into the cleaning cover 104 is drained to the outside of the cleaning cover 104 via the drain ports 125. The drain ports 125 are formed in the respective wall portions of the cleaning cover 104 surrounding the cleaning object. Therefore, even when the cleaning cover 104 is disposed in any arrangement, the cleaning water can be drained to the outside of the cleaning cover 104 through any drain port 125. Therefore, the cleaning water does not remain in the cleaning cover 104, and the cleaning efficiency is enhanced.

In the cleaning, the cleaning device 100 together with the attached ultrasonic treatment instrument 1 is disposed in the cleaning tank of the cleaning machine or in the cleaning tray of the cleaning unit, and the exterior portion of the ultrasonic treatment instrument 1 is preferably simultaneously cleaned by the shower cleaning or by the exposure into the running water.

The respective components of the cleaning device 100 can all be disassembled, when the connected portion of the screw-in portion is removed. It is easy to clean these components.

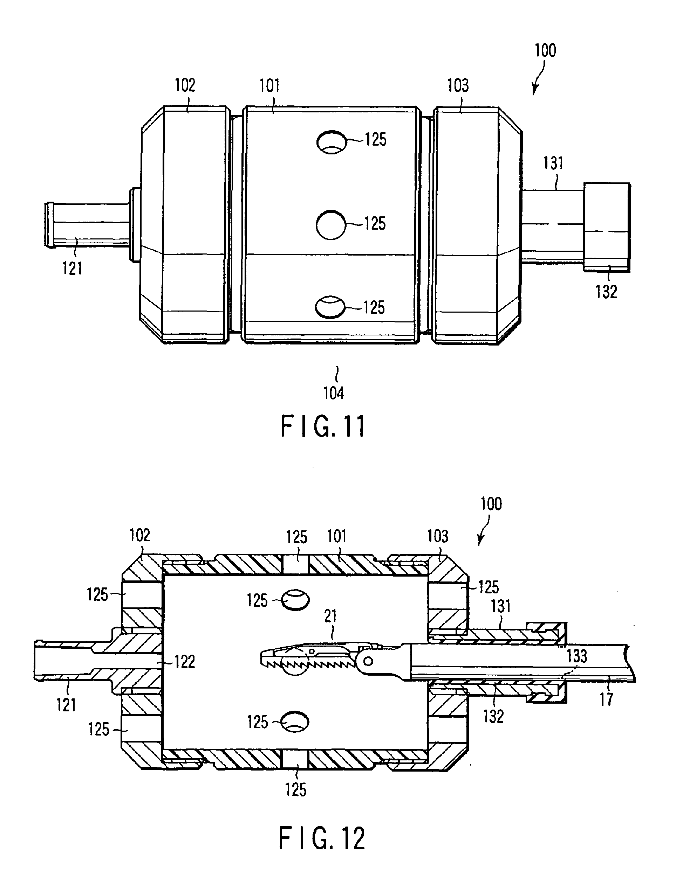

The cleaning device according to a third embodiment of the present invention will be described with reference to FIGS. 11 and 12. In the present embodiment, the same components as those of the first and second embodiments are denoted with the same reference numerals, and the detailed description thereof is omitted.

The cleaning device 100 of the present embodiment is different from that of the second embodiment only in the fixing means held in the surgical treatment instrument. That is, an elastic tube 132 is disposed so as to be closely fitted over the outer periphery of the proximate-end portion of a hold cylinder 131 from an inner surface of the hold cylinder 115, and a rubber member forming the elastic tube 132 is inner-molded with respect to the hold cylinder 131. A rib 133 which abuts on the outer periphery of the insertion sheath portion 17 is disposed on the inner surface of the elastic tube 132, and an engaging force with respect to the insertion sheath portion 17 is enhanced.

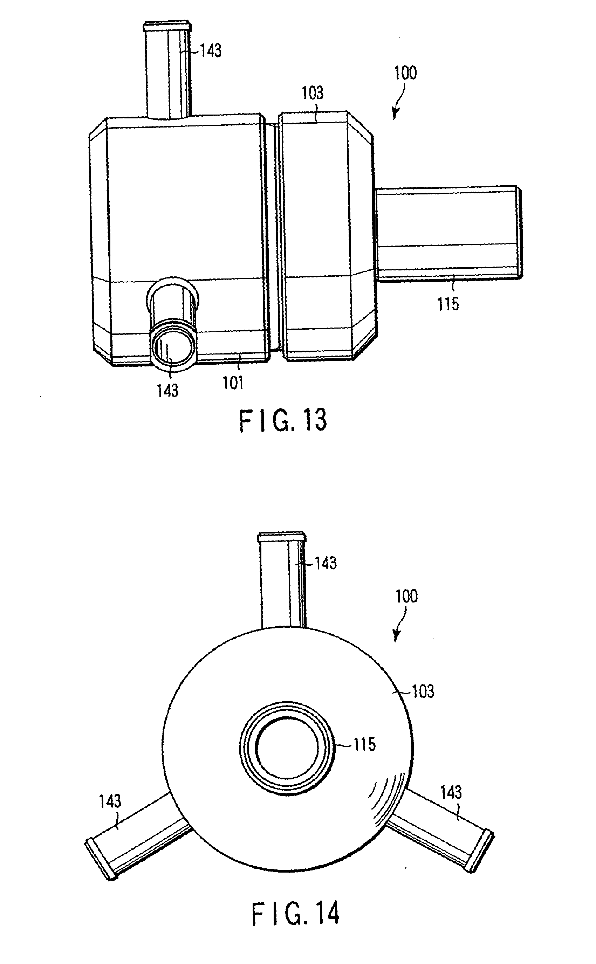

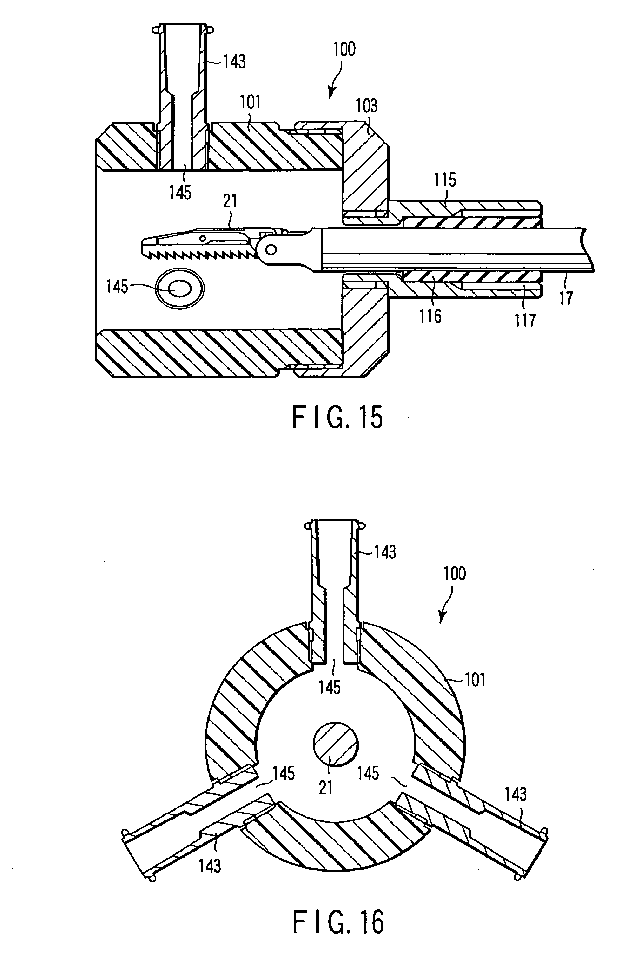

The cleaning device according to the third embodiment of the present invention will be described with reference to FIGS. 13 to 16. For the cleaning device 100 according to the present embodiment, the front-end plate 102 in the cleaning device according to the second embodiment is removed, the front-end opening of the cylindrical member 101 is opened, and the front-end opening is used as a drain port 141 to constitute a cleaning cover 142. The drain ports 125 in the cleaning device 100 according to the second embodiment are not disposed. A plurality of supply nozzles 143 are disposed in the cylindrical member 101 of the cleaning cover 142, and inner ends of the supply nozzles 143 form a jet ports (water supply ports) 145 in the inner surface of the cylindrical member 101. Any of the respective jet ports 145 surrounds the cleaning region where the cleaning object is disposed in the cleaning cover 142, and the jet port is disposed so as to be directed to the cleaning region from the periphery. Here, three supply nozzles 143 are disposed, and the supply nozzles 143 are arranged so that the water is equally radiated in three directions from the center of the cleaning cover 142.

When the cleaning device 100 of the present embodiment is used, the high-pressure cleaning water is spouted toward the distal-end function portion 21 of the cleaning object disposed in the cleaning cover 104 via three jet ports 145. Moreover, the cleaning is possible in which the cleaning water is spouted from three peripheral directions. Therefore, even the strong dirt can be removed, and every corner can strongly be cleaned without any unevenness.

The cleaning water which has overflowed in the cleaning cover 104 can be drained to the outside of the cleaning cover 104 from the drain port 141 which largely opens forwards. The other constitution is similar to that of the cleaning device according to the above-described embodiments.

As described above, according to the cleaning device of the present invention, for the medical instrument, especially the treatment portion which easily strongly becomes dirty can efficiently be cleaned.

## Claims

1. A cleaning device which cleans a treatment portion (21) of a medical instrument (1), comprising:
a cleaning cover (81) including a containing portion (88) which surrounds the treatment portion (21) and which contains the treatment portion (21), and an insertion opening (86) via which the treatment portion (21) is inserted into the containing portion (88);
an engagement portion (92, 116, 132) which engages with the medical instrument (1) including the treatment portion (21) inserted in the containing portion (88) via the insertion opening (86) and which holds the medical instrument (1) in the cleaning cover (81);
a water supply port (91) which is disposed in the cleaning cover (81) and which communicates with the containing portion (88) and which supplies cleaning water into the containing portion (88); and
a drain port (90) which is disposed in the cleaning cover (81) and which communicates with the containing portion (88) and via which the cleaning water in the containing portion (88) is drained to the outside of the cleaning cover (81);
**characterized in that** the engagement portion (92, 116, 132) comprises an elastic tube member arranged in the insertion opening (86), the elastic tube member being elastically deformable to engage with the medical instrument (1) when the treatment portion (21) is inserted into the containing portion (88), such that the engagement portion (92, 116, 132) contacts the outer periphery of the treatment portion (21) with pressure to fix the treatment portion (21) within the containing portion (88) and to seal the insertion opening (86).

2. The cleaning device for the medical instrument (1) according to claim 1, **characterized in that** the water supply port (91) is provided on an outer wall of the containing portion (88), and which further includes a water supply nozzle (87) which is formed in an inner wall of the containing portion (88) and which spouts the cleaning water toward the treatment portion (21) contained in the containing portion (88).

3. The cleaning device for the medical instrument according to claim 1 or 2, **characterized by** further includes a plurality of water supply nozzles (87) which are formed in an inner wall of the containing portion and which spout the cleaning water toward the treatment portion (21) contained in the containing portion (88), and the water supply nozzles (87) are arranged over a whole periphery in a peripheral direction of an inner surface of the containing portion (88).

4. The cleaning device for the medical instrument according to claim 3, **characterized in that** the containing portion (88) is constituted of two cylindrical members (61, 62) arranged coaxially, and
further includes a water supply channel (89) which is formed by a gap formed between both the cylindrical members (61, 62) and which connects the water supply port (91) to the water supply nozzles (87).

5. The cleaning device for the medical instrument according to claim 1, 2, or 3, **characterized in that** the cleaning cover (81) is formed of a transparent material via which the containing portion (88) able to be seen through.

6. The cleaning device according to any one of claims 1 to 5, wherein the elastic tube member of the engagement portion (92, 116, 132) is coaxially arranged within a rigid tube member (115, 131) such that the elastic tube defines the insertion opening (86).

7. The cleaning device according to claim 6, further comprising a ring member (95, 117) adapted to be screwed into the rigid tube member (115, 131) to compress the elastic tube member in the axial direction so that an inner diameter of the elastic tube member can be reduced.

8. The cleaning device according to any one of claims 1 to 6, wherein the elastic tube member includes a radially inwardly projecting rib (133) disposed on an inner surface of the elastic tube member, wherein the rib (133) is adapted to enhance an engaging force with respect to the treatment portion (21) of the medical instrument.

9. The cleaning device according to any one of the preceding claims, wherein the water supply port (122) is formed at an end of the containing portion (88) opposite the insertion opening (86).

10. The cleaning device according to any one of the preceding claims comprising a plurality of drain ports (125) formed in wall portions of the cleaning cover (81) which surround the treatment portion (21).

## Patentansprüche

1. Reinigungsvorrichtung, die einen Behandlungsabschnitt (21) eines medizinischen Instruments (1) reinigt, umfassend:
eine Reinigungsabdeckung (81) mit einem Aufnahmeabschnitt (88), der den Behandlungsabschnitt (21) umschließt und der den Behandlungsabschnitt (21) aufnimmt, und mit einer Einführungsöffnung (86), über die der Behandlungsabschnitt (21) in den Aufnahmeabschnitt (88) eingeführt wird;
einen Eingriffsabschnitt (92, 116, 132), der mit dem medizinischen Instrument (1) einschließlich des Behandlungsabschnitts (21), der in den Aufnahmeabschnitt (88) über die Einführungsöffnung (86) eingeführt ist, in Eingriff kommt und der das medizinische Instrument (1) in der Reinigungsabdeckung (81) hält;
eine Wasserzuführöffnung (91), die in der Reinigungsabdeckung (81) vorgesehen ist und die mit dem Aufnahmeabschnitt (88) in Verbindung steht und durch welche Reinigungswasser in den Aufnahmeabschnitt (88) eingegeben wird, und
eine Abflussöffnung (90), die in der Reinigungsabdeckung (81) vorgesehen ist und die mit dem Aufnahmeabschnitt (88) in Verbindung steht und über die das Reinigungswasser in den Aufnahmeabschnitt (88) zur Außenseite der Reinigungsabdeckung (81) abgelassen wird;
**dadurch gekennzeichnet, dass** der Eingriffsabschnitt (92, 116, 132) ein elastisches Rohrstück umfasst, das in der Einführungsöffnung (86) angeordnet ist, und das elastische Rohrstück elastisch verformbar ist, um mit dem medizinischen Instrument (1) in Eingriff zu kommen, wenn der Behandlungsabschnitt (21) in den Aufnahmeabschnitt (88) eingeführt ist, derart, dass der Eingriffsabschnitt (92, 116, 132) an der äußeren Umfangsfläche des Behandlungsabschnitts (21) unter Druck anliegt, um den Behandlungsabschnitt (21) innerhalb des Aufnahmeabschnitts (88) zu fixieren und die Einführungsöffnung (86) zu verschließen.

2. Reinigungsvorrichtung für das medizinische Instrument (1) gemäß Anspruch 1, **dadurch gekennzeichnet, dass** die Wasserzuführöffnung (91) in einer Außenwand des Aufnahmeabschnitts (88) vorgesehen ist, die ferner eine Wasserzufuhrdüse (87) aufweist, die in einer Innenwand des Aufnahmeabschnitts (88) ausgebildet ist und die das Reinigungswasser zum Behandlungsabschnitt (21), der in dem Aufnahmeabschnitt (88) aufgenommen ist, hin spritzt.

3. Reinigungsvorrichtung für das medizinische Instrument nach Anspruch 1 oder 2 **dadurch gekennzeichnet, dass** es ferner mehrere Wasserzufuhrdüsen (87) aufweist, die an der Innenwand des Aufnahmeabschnitts ausgebildet sind und das Reinigungswasser auf den Behandlungsabschnitt (21), der in dem Aufnahmeabschnitt (88) aufgenommen ist, spritzen, und dass die Wasserzufuhrdüsen (87) in Umfangsrichtung über den ganzen Umfang der Innenfläche des Aufnahmeabschnitts (88) verteilt angeordnet sind.

4. Reinigungsvorrichtung für das medizinische Instrument nach Anspruch 3, **dadurch gekennzeichnet, dass** der Aufnahmeabschnitt (88) aus zwei zueinander koaxial angeordneten zylindrischen Teilen (61, 62) gebildet ist, und ferner einen Wasserzufuhrkanal (89) aufweist, der durch einen Spalt zwischen den beiden zylindrischen Teilen (61, 62) gebildet ist, der die Wasserzufuhröffnung (91) mit den Wasserzufuhrdüsen (87) verbindet.

5. Reinigungsvorrichtung für das medizinische Instrument nach den Ansprüchen 1, 2 oder 3, **dadurch gekennzeichnet, dass** die Reinigungsabdeckung (81) aus einem transparenten Werkstoff geformt ist, über den der Aufnahmeabschnitt (88) hindurch gesehen werden kann.

6. Reinigungsvorrichtung nach einem der Ansprüche 1 bis 5, bei dem das elastische Rohrteil des Eingriffsabschnitts (92, 116, 132) koaxial zu einem starren Rohrteil (115, 131) derart angeordnet ist, dass das elastische Rohr die Einführungsöffnung (86) bildet.

7. Reinigungsvorrichtung nach Anspruch 6, die ferner ein Ringstück (95, 117) aufweist, das in das starre Rohrteil einschraubbar ist (115, 131), um das elastische Rohrteil in axialer Richtung zu stauchen, so dass der Innendurchmesser des elastischen Rohrteils verkleinert werden kann.

8. Reinigungsvorrichtung nach einem der Ansprüche 1 bis 6, bei der das elastische Rohrteil eine radial nach innen vorspringende Rippe (133) hat, die an der Innenfläche des elastischen Rohrteils vorgesehen ist, und die Rippe (133) die Eingriffskraft auf den Behandlungsabschnitt (21) des medizinischen Instruments erhöhen kann.

9. Reinigungsvorrichtung nach einem der vorhergehenden Ansprüche, bei der die Wasserzufuhröffnung (122) an dem einen Ende des Aufnahmeabschnitts (88) gegenüber der Einführungsöffnung (86) ausgebildet ist.

10. Reinigungsvorrichtung nach einem der vorhergehenden Ansprüche, die mehrere Abflussöffnungen (125) aufweist, die in einem Wandabschnitt der Reinigungsabdeckung (81) ausgebildet sind, die den Behandlungsabschnitt (21) umschließt.

## Revendications

1. Dispositif de nettoyage qui nettoie une partie de traitement (21) d'un instrument médical (1), comprenant :
une enveloppe de nettoyage (81) incluant une partie de confinement (88) qui entoure la partie de traitement (21) et qui confine la partie de traitement (21), et une ouverture d'introduction (86) via laquelle on introduit la partie de traitement (21) dans la partie de confinement (88) ;
une partie d'engagement (92, 116, 132) qui engage avec l'instrument médical (1) incluant la partie de traitement (21) introduite dans la partie de confinement (88) via l'ouverture d'introduction (86) et qui maintient l'instrument médical (1) dans l'enveloppe de nettoyage (81) ;
un orifice d'alimentation en eau (91) qui est disposé dans l'enveloppe de nettoyage (81) et qui communique avec la partie de confinement (88) et qui alimente en eau la partie de confinement (88) ; et
un orifice de drainage (90) qui est disposé dans l'enveloppe de nettoyage (81) et qui communique avec la partie de confinement (88) et via laquelle l'eau de nettoyage qui se trouve dans la partie de confinement (88) est drainée à l'extérieur de l'enveloppe de nettoyage (81) ;
**caractérisé en ce que** la partie d'engagement (92, 116, 132) comprend un élément de tube élastique agencé dans l'ouverture d'introduction (86), l'élément de tube élastique pouvant se déformer élastiquement pour engager avec l'instrument médical (1) lorsque la partie de traitement (21) est introduite dans la partie de confinement (88), de sorte que la partie d'engagement (92, 116, 132) contacte la périphérie extérieure de la partie de traitement (21) avec une certaine pression pour fixer la partie de traitement (21) à l'intérieur de la partie de confinement (88) et pour fermer hermétiquement l'ouverture d'introduction (86).

2. Dispositif de nettoyage prévu pour l'instrument médical (1) selon la revendication 1, **caractérisé en ce que** l'orifice d'alimentation en eau (91) est réalisé sur une paroi extérieure de la partie de confinement (88), et qui inclut en outre une buse de distribution d'eau (87) qui est formée dans une paroi intérieure de la partie de confinement (88) et qui décharge l'eau de nettoyage en direction de la partie de traitement (21) confinée dans la partie de confinement (88).

3. Dispositif de nettoyage prévu pour l'instrument médical selon la revendication 1 ou 2, **caractérisé par le fait qu'**il comprend en outre une pluralité de buses de distribution d'eau (87) qui sont formées dans une paroi intérieure de la partie de confinement et qui déchargent l'eau de nettoyage en direction de la partie de traitement (21) confinée dans la partie de confinement (88), et en ce que les buses de distribution d'eau (87) sont agencées sur toute la périphérie d'une surface intérieure de la partie de confinement (88).

4. Dispositif de nettoyage prévu pour l'instrument médical selon la revendication 3, **caractérisé en ce que** la partie de confinement (88) est constituée de deux éléments cylindriques (61, 62) agencés coaxialement, et
**en ce qu'**il inclut en outre un canal d'alimentation en eau (89) qui est formé par un espace formé entre les deux éléments cylindriques (61, 62) et qui établit une communication entre l'orifice d'alimentation en eau (91) et les buses de distribution d'eau (87).

5. Dispositif de nettoyage prévu pour l'instrument médical selon la revendication 1, 2, ou 3, **caractérisé en ce que** l'enveloppe de nettoyage (81) est formée à partir d'un matériau transparent à travers lequel on peut observer la partie de confinement (88).

6. Dispositif de nettoyage selon l'une quelconque des revendications 1 à 5, dans lequel l'élément de tube élastique de la partie d'engagement (92, 116, 132) est agencé coaxialement à l'intérieur d'un élément de tube rigide (115, 131) de sorte que le tube élastique définit l'ouverture d'introduction (86).

7. Dispositif de nettoyage selon la revendication 6, comprenant en outre un élément annulaire (95, 117) adapté pour vissage dans l'élément de tube rigide (115, 131) à des fins de comprimer l'élément de tube élastique dans la direction axiale pour pouvoir réduire un diamètre intérieur de l'élément de tube élastique.

8. Dispositif de nettoyage selon l'une quelconque des revendications 1 à 6, dans lequel l'élément de tube élastique inclut une nervure faisant saillie radialement vers l'intérieur (133) disposée sur une surface intérieure de l'élément de tube élastique, dans lequel la nervure (133) est adaptée pour améliorer une force d'engagement par rapport à la partie de traitement (21) de l'instrument médical.

9. Dispositif de nettoyage selon l'une quelconque des revendications précédentes, dans lequel l'orifice d'alimentation en eau (122) est formé au niveau d'une extrémité de la partie de confinement (88) opposée à la partie d'introduction (86).

10. Dispositif de nettoyage selon l'une quelconque des revendications précédentes, comprenant une pluralité d'orifices de drainage (125) formés dans des parties de paroi de l'enveloppe de nettoyage (81) qui entoure la partie de traitement (21).
